# EUROPEAN PATENT SPECIFICATION

(11) **EP 2 698 122 B1**
(45) Date of publication and mention of the grant of the patent: **05.07.2017**
(21) Application number: 13188756.4
(22) Date of filing: 29.04.2010
(51) Int. Cl.: A61B 17/80, A61B 17/17

(54) **Orthognathic implant**
Orthognatisches Implantat
Implant orthognatique

(43) Date of publication of application: 19.02.2014
(62) Divisional of application: 10717954.1
(73) Proprietor: Synthes GmbH, 4436 Oberdorf (CH)
(72) Inventor: Furrer, Andre, 4436 Oberdorf (CH); Zillig, Timo, 4313 Moehlin (CH); Metzger, Marc Christian, 79106 Freiburg (DE)
(74) Representative: Klunker IP Patentanwälte PartG mbB

(56) References cited:
- EP-A1- 1 654 994
- WO-A2-2008/112074
- GB-A- 2 324 470
- US-A1- 2002 128 654

## Description

### BACKGROUND

Orthognathic surgery is generally performed to correct conditions of the jaw and face related to structure, growth, sleep apnea, TMJ disorders or to correct orthodontic problems. For example, an individual who has a significantly receded upper jaw or an open bite might benefit from a maxillary osteotomy. In such a procedure, a surgeon makes cuts below both eye sockets to separate a segmented part of the maxilla from an intact portion of the maxilla. The entire segmented part, including the roof of the mouth and all upper teeth, can move as a single unit. The segmented part is then moved until the upper and bottom teeth fit together properly. Once the teeth are realigned, tiny screws and plates are used to fix the segmented part of the maxilla in its new position until natural bone healing takes place.

Some orthognathic surgeries affix multiple plates to the maxilla to hold the cut segmented part of the maxilla relative to the second intact part. As one could imagine, the adaptation and use of multiple plates make the procedure unnecessarily long and complicated.

Other plating systems require multiple disciplines such as surgeons, dentists, orthodontists, etc to complete the procedure. As a result there often times are misunderstandings between the disciplines. These and other disadvantages are attributed to such plating systems used in orthognathic surgeries. GB 2 324 470 A and WO 2008/112074 A2 disclose bone plates for the treatment of fractures, in particular for the reconstruction of mandibular fractures. US 2002/0128654 A1 and EP 1 654 994 A1 disclose bone plates for fixation of the sternum. The bone plates have first and second portions connected by a bridge portion. The bridge portion can be cut in order to separate the first and second portions of the bone plate.

Therefore, it may be desired to achieve a better and more accurate way of planning and performing orthognathic surgery.

### SUMMARY

The disclosure generally relates to an improvement in implants used in orthognathic surgery, and in particular, patient specific plates for use in orthognathic surgery. However, the disclosed implants are not limited to this specific application.

In one embodiment, an implant for use in orthognathic surgery may include a plate member and a plurality of fingers extending out from the plate member. The plate member is pre-shaped to correspond to a pre-operative shape of a maxilla. The plate member includes at least one fixation aperture that extends through the plate member and is configured to receive a bone fixation element so as to secure the plate member to the maxilla. The fingers are pre-shaped to correspond to the shape of the maxilla.

It is further disclosed an implant that is configured to join a first part of the maxilla to a second part of the maxilla after a segmentation procedure that separates the first part of the maxilla from the second part of the maxilla is disclosed. The implant includes a plate member and a holding structure that extends from the plate member. The plate member is pre-shaped, prior to the segmentation procedure, so as to correspond to an outer surface of the first part of the maxilla after the segmentation procedure. The plate member includes at least one aperture configured to receive a fixation element so as to secure the plate member to the first part of the maxilla. The holding structure is pre-shaped so as to correspond to the second part of the maxilla after the segmentation procedure.

It is further disclosed an osteotomy guiding implant for use in orthognathic surgery is disclosed. The osteotomy guiding implant may include a plate member and a plurality of fingers that extend from the plate member. The plate member and the fingers are pre-shaped to correspond to a pre-operative shape of a maxilla. Each finger defines an aperture, such that the apertures of the fingers are arranged to provide a template for pre-osteotomy drilling holes that define a cutting guide path on the maxilla so as to separate a segmented portion of the maxilla from an integral portion of the maxilla.

In an embodiment according to the invention, the implant includes a plate member having a first portion, and a second portion separated by a bridge portion. The first and second portions each include at least one aperture configured to receive a fixation element so as to secure the plate member to a maxilla. At least one finger extends from each of the first and second portions, wherein each finger includes at least one aperture configured to receive a fixation element so as to secure each finger to the maxilla. After the plate member has been secured to the maxilla, the bridge portion is removable to thereby separate the first portion of the plate member from the second portion of the plate member.

A method for correcting the shape of a maxilla, is also disclosed. Preferably a plurality of locations on the maxilla at which a plurality of holes are to be located is determined. A guiding implant is positioned on the maxilla such that guiding apertures of the guiding implant are arranged to align with the plurality of locations. Holes are then made in the maxilla using the guiding apertures of the guiding implant. Based on the location of the holes, an osteotomy is performed to separate the maxilla into at least a first part and a second part. Once the osteotomy is completed, a pre-shaped bone fixation implant is positioned on the maxilla and is arranged to hold the maxilla in a corrected shape. The bone fixation implant is pre-shaped to correspond to the post-operative shape of the maxilla. Once in place, the bone fixation implant is affixed to the maxilla using fixation elements.

A method of customizing a pre-shaped implant for use in orthognathic surgery of a maxilla is also disclosed. To customize the implant a pre-operative 3-D model of a patient's maxilla is first obtained in a computer whereby the first portion of the maxilla and the second portion of the maxilla define first relative position. The pre-operative 3-D model of the maxilla is then manipulated into a post-operative shape, whereby the first portion of the maxilla and the second portion of the maxilla define a second relative position that is different than the first relative position. Once in the desired position, a bone fixation implant is custom constructed to match the planned post-operative shape of the maxilla. The implant may include a longitudinal plate member and a plurality of fingers that extend from an upper edge of the plate member.

### BRIEF DESCRIPTION OF THE DRAWINGS

The foregoing summary, as well as the following detailed description of example embodiments, are better understood when read in conjunction with the appended diagrammatic drawings. For the purpose of illustrating the invention, the drawings show embodiments that are presently preferred. The invention is not limited, however, to the specific instrumentalities disclosed in the drawings.
Fig. 1 is a perspective view of a skull with a bone fixation implant affixed to the maxilla;
Fig. 2A is a perspective view of a bone fixation implant constructed in accordance with one embodiment;
Fig. 2B is a front view of the bone fixation implant shown in Fig. 2A;
Fig. 2C is a top view of the bone fixation implant shown in Fig. 2A;
Fig. 2D is a left side view of the bone fixation implant shown in Fig. 2A;
Fig. 3A is a perspective view of an osteotomy guiding implant constructed in accordance with one embodiment;
Fig. 3B is a front view of the osteotomy guiding implant shown in Fig. 3A;
Fig. 3C is a top view of the osteotomy guiding implant shown in Fig. 3A;
Fig. 3D is a left side view of the osteotomy guiding implant shown in Fig. 3A;
Fig. 4 is a diagram showing the process for customizing the bone fixation implant of Figs. 2A-2D and the osteotomy guiding implant of Figs. 3A-3D to correspond to an individual patient's maxilla;
Fig. 5A is a front view of a skull, including a maxilla bone that is to be operated on;
Fig. 5B is an enlarged detailed view showing the pre-operative shape of the maxilla of the skull shown in Fig. 5A;
Fig. 5C is a front view of the maxilla shown in Fig. 5B, showing the osteotomy guiding implant of Figs. 3A-3D being attached to the maxilla;
Fig. 5D is a front view of the of the maxilla shown in Fig. 5C, showing holes being drilled into the maxilla through guide holes defined by the osteotomy guiding implant;
Fig. 5E is a front view of the maxilla shown in Fig. 5D, showing the drilled holes;
Fig. 5F is a front view of the maxilla shown in Fig. 5E, showing the osteotomy performed on the maxilla, using the holes as a cutting guide;
Fig. 5G is a front view of the maxilla shown in Fig. 5F, showing a segmented portion of the maxilla being repositioned into a post-operative shape;
Fig. 5H is a front view of the maxilla shown in Fig. 5G, showing the bone fixation implant of Figs. 2A-2D being attached to the maxilla;
Fig. 5I is a front view of the maxilla shown in Fig. 5H, showing the bone fixation implant attached to the maxilla; and
Fig. 5J is a front view of the maxilla shown in Fig. 5I, showing a bridge portion of the bone fixation implant removed.

### DETAILED DESCRIPTION OF ILLUSTRATIVE EMBODIMENTS

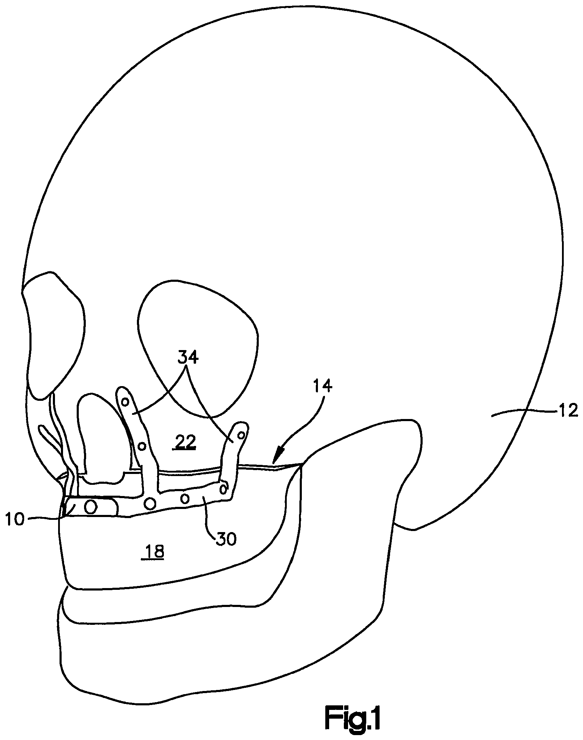

Referring to Fig. 1 a bone fixation implant 10 to be used in orthognathic surgery is designed to be fixed to underlying bone such as a patient's skull 12, and in particular to a patient's maxilla 14 after the maxilla 14 has been separated into a first "segmented" part 18 and a second "integral" part 22 by a segmentation procedure, such as an osteotomy. The first part 18 of the maxilla 14 typically carries the upper teeth and is completely separated from the skull 12 after the osteotomy has been performed, while the second part 22 of the maxilla 14 remains intact with the skull 12. The bone fixation implant 10 is configured to attach to the first and second parts of the maxilla, and thereby support and hold the first part 18 of the maxilla relative to the second part 22 while osteogenesis occurs. The implant 10 is customized pre-operatively to minimize complications during surgery and time spent in the operating room by a patient.

Certain terminology is used in the following description for convenience only and is not limiting. The words "right", "left", "lower" and "upper" designate directions in the drawings to which reference is made. The words "inner" or "distal" and "outer" or "proximal" refer to directions toward and away from, respectively, the geometric center of the implant and related parts thereof. The words, "anterior", "posterior", "superior," "inferior," "medial," "lateral," and related words and/or phrases designate preferred positions and orientations in the human body to which reference is made and are not meant to be limiting. The terminology includes the above-listed words, derivatives thereof and words of similar import.

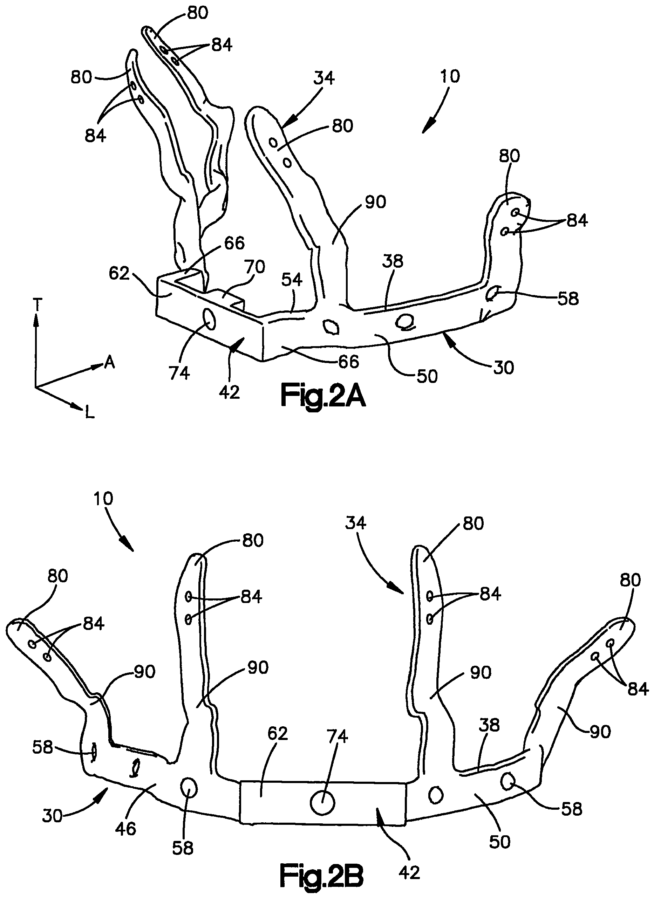

Referring to Figs. 2A-2D, the implant 10 and various components of the implant are described herein extending horizontally along a longitudinal direction "L" and lateral direction "A", and vertically along a transverse direction "T". Unless otherwise specified herein, the terms "lateral," "longitudinal," and "transverse" are used to describe the orthogonal directional components of various components. When the implant 10 is implanted onto a maxilla, such as the maxilla 14, the transverse direction T extends vertically generally along the superior-inferior (or caudal-cranial) direction, while the plane defined by the longitudinal direction L and lateral direction A extends horizontally, generally in the anatomical plane defined by the medial-lateral direction and the anterior-posterior direction. Accordingly, the directional terms "vertical" and "horizontal" are used to describe the implant 10 and its components as illustrated merely for the purposes of clarity and illustration.

As shown in Figs. 2A-2D, the bone fixation implant 10 includes a longitudinal plate member 30 that is elongate and curved in the longitudinal direction L and a holding structure 34 that extends vertically from the longitudinal member 30. The longitudinal plate member 30 includes an upper edge 38, a bone engaging surface configured to lie substantially flush with the maxilla, and an outer surface opposed to the bone engaging surface. Therefore, the holding structure 34 extends up from the upper edge 38 of the longitudinal member 30. As shown in Fig. 1, the bone fixation implant 10 supports and holds the first part 18 of the maxilla relative to the second part 22 while osteogenesis occurs. The bone fixation implant 10 and components thereof, can be formed from a variety of biocompatible materials, such as cobalt chromium molybdenum (CoCrMo), titanium, and titanium alloys, stainless steel, ceramics, or polymers such as polyetheretherketone (PEEK), polyetherketoneketone (PEKK), and bioresorbable materials. A coating may be added or applied to the bone fixation implant 10 to improve physical or chemical properties or to provide medications. Examples of coatings include plasma-sprayed titanium coating or Hydroxyapatite.

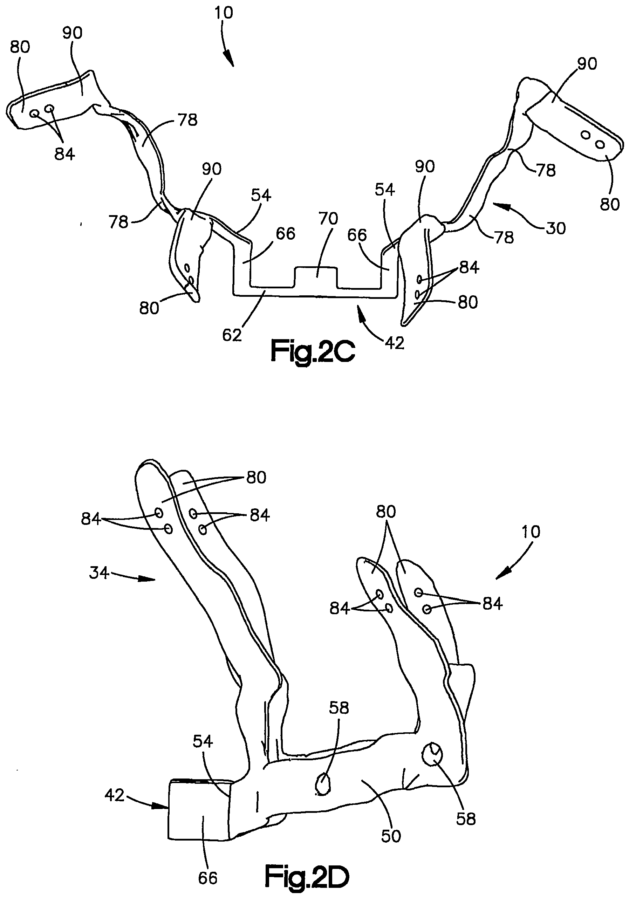

As shown in Figs. 1, and 2A-2D, the longitudinal member 30 is configured to be attached to the first part 18 of the maxilla 14. In general, the longitudinal member 30 includes a central bridge member 42 that separates the longitudinal member 30 into a first portion 46 and a second portion 50. The first and second portions 46, 50 extend from the bridge member 42 from respective junctions 54. As shown, the first portion 46 extends from the bridge member 42 in a first direction, while the second portion 50 extends from the bridge member 42 in a second direction that is generally opposite to the first direction. As best shown in Fig. 2C, the first portion 46 and the second portion 50 each curves in the lateral direction A as they extend longitudinally. Therefore, as best shown in Fig. 2C, the longitudinal member 30 is curved such that it forms generally a C-shaped structure. Furthermore, as best shown in Figs. 2B and 2D, the first portion 46 and the second portion 50 each angle up in the transverse direction T as they extend longitudinally. The curvature and shape of the longitudinal member 30 generally correspond to the shape of the maxilla 14.

Additionally, the first and second portions 46, and 50 of the longitudinal member 30 include a plurality of fixation element receiving apertures/holes 58 that extend from the outer surface of the longitudinal member 30 and through to the bone engaging surface. Each hole 58 is configured to receive a fixation element, such as a screw. Though it should be understood that any fixation element will suffice. The implant 10 is configured to be fastened to the first part 18 of the maxilla 14 by inserting fixation elements through each hole 58 of the longitudinal member 30 and into the first part 18 of the maxilla 14.

The bridge member 42 of the longitudinal member 30 includes a plate 62 that is elongate in the longitudinal direction L, an extension 66 extending in the lateral direction A from each end of the plate 62, and a centrally located protrusion 70 that also extends laterally from an inner surface of the plate 62. The junctions 54 are located at the posterior ends of each extension 66. Thus, the first and second portions 46, 50 of the longitudinal member 30 each extend from a posterior end of a respective extension 66 of the bridge member 42. The bridge member 42 may be removed from the longitudinal member 30 at the junctions 54 once the implant 10 is secured to the maxilla 14. The junction points 54 may be weakened so that the bridge member 42 may be easily removed once the implant 10 is secured to the maxilla 14. For example, junctions 54 may be thinned, or perforated, or otherwise configured, so that the bridge member 42 may be removed by snapping the bridge member 42 away. It should be understood however that the bridge member 42 may be removed by cutting the junction points 54 with snips or pliers. Because the bridge member 42 is removable, the amount of the implant 10 left in the patient may be minimized.

As shown in Fig. 2B, the bridge member further includes a reference hole 74 that extends laterally through both the plate 62 and the protrusion 70 of the bridge member 42. The implant 10 may initially be fastened to the maxilla 14 by inserting a fixation element through the reference hole 74 and into the maxilla 14. The fixation element inserted into the reference hole 74 may temporarily affix the implant 10 to the maxilla 14 while a surgeon correctly aligns the implant 10 for complete fixation to the maxilla 14.

The longitudinal member 30 and in particular the first and second portions 46, 50, is pre-shaped to correspond to the post-operative shape of the first part 18 of the maxilla 14. In this regard the longitudinal member is pre-shaped prior to the segmentation procedure, so as to correspond to an outer surface of the first part of the maxilla after the segmentation procedure. While it is preferable that the member 30 is pre-shaped such that no manual bending is required prior to placement of the implant 10 onto the maxilla 14, the member 30 may be pre-shaped such that only minimal bending is required prior to placement of the implant 10 onto the maxilla 14 (e.g. bending that may take place when fastening the member 30 to the maxilla 14). As best shown in Fig. 2C, the first and second portions 46, 50 include several non-linear undulations 78 that correspond to particular surface portions of the first part 18 of the maxilla 14. It should be understood, however, that the shape of the first part 18 of the maxilla 14 may be unchanged between the pre-operative and post-operative shape of the maxilla 14. Therefore, the longitudinal member 30 may be pre-shaped to correspond to both the pre-operative shape and the post-operative shape of the first part 18 of the maxilla 14.

As shown in Figs. 2A-2D, the holding structure 34 of the implant 10 includes at least one finger 80, such as a plurality of fingers 80 that extend up from the upper edge 38 of the longitudinal member 30. In accordance with the illustrative embodiment, two fingers 80 extend from each of the first and second portions 46, and 50 of the longitudinal member 30. However, it should be understood that any number of fingers 80 may extend up from the first and second portions 46, and 50. As shown, each finger 80 includes at least one fixation element receiving aperture or hole 84 configured to receive a fixation element, such as a screw, so as to affix the fingers 80 to the second part 22 of the maxilla 14. Though it should be understood any fixation element will suffice. While the embodiment illustrated shows each finger 80 having two holes 84, it should be understood, that each finger may have any number of holes, e.g. 1, 2, 3, 4, etc.

As best shown in Fig. 2B, the fingers 80 are spaced apart along the first and second portions 46, 50 of the longitudinal member 30 and extend substantially perpendicularly relative to the point on the portions 46, 50 from where they extend. That is, the longitudinal plate member 30 is non-linear and will define tangents at different points along its edge 38. Therefore, each finger 80 will extend perpendicular with respect to a tangent taken at the point on the edge 38 from which the finger 80 extends. Though it should be understood that the fingers 80 do not have to extend perpendicularly, and may extend at some angle relative to the longitudinal member 30. Preferably, each finger 80 extends from the longitudinal member 30 such that a fixation element hole 58 of the longitudinal member 30 is aligned with the point at which a respective finger 80 extends from the edge 38 of the longitudinal member 30, to further improve equal force distribution throughout the implant 10.

The holding structure 34 or fingers 80 are pre-shaped to correspond to the post-operative shape of the second part 22 of the maxilla 14, and extend from the first and second portions 46, 50, so as to provide a fixation member that corresponds to the shape and the relationship of the first parts of the maxilla. In this regard the fingers 80 are pre-shaped prior to the segmentation procedure, so as to correspond to an outer surface of the second part of the maxilla after the segmentation procedure. While it is preferable that the fingers 30 are pre-shaped such that no manual bending is required prior to placement of the implant 10 onto the maxilla 14, the fingers 80 may be pre-shaped such that only minimal bending is required prior to placement of the implant 10 onto the maxilla 14. Therefore, as best shown in Fig. 2C, the fingers 80 include several non-linear undulations 90 that correspond to particular surface portions of the second part 22 of the maxilla 14. Because the fingers 80 are pre-shaped, they will fit correctly only at the desired location of the maxilla 14 and provide a surgeon with positive assurance that they have achieved correct alignment and, therefore, a desired corrected shape.

Before the implant 10 is affixed to the maxilla, an osteotomy is performed to separate the maxilla 14 into the first part 18 and the second part 22. A temporary osteotomy guiding implant 110 may be affixed to the maxilla 14 before the osteotomy is performed on the maxilla 14 to create a guide for the surgeon. In particular, the osteotomy guiding plate 110 provides a template for a surgeon to follow while performing the osteotomy. For example, the osteotomy guiding implant 110 allows the surgeon to make guide holes in the maxilla to follow while performing the osteotomy. In this way, the osteotomy guiding implant acts as a drill guiding implant. The osteotomy guiding implant also provides a template for the surgeon to follow while implanting the bone implant 10. The osteotomy guiding implant 110 is also customized pre-operatively to minimize complications during surgery and time spent in the operating room by a patient.

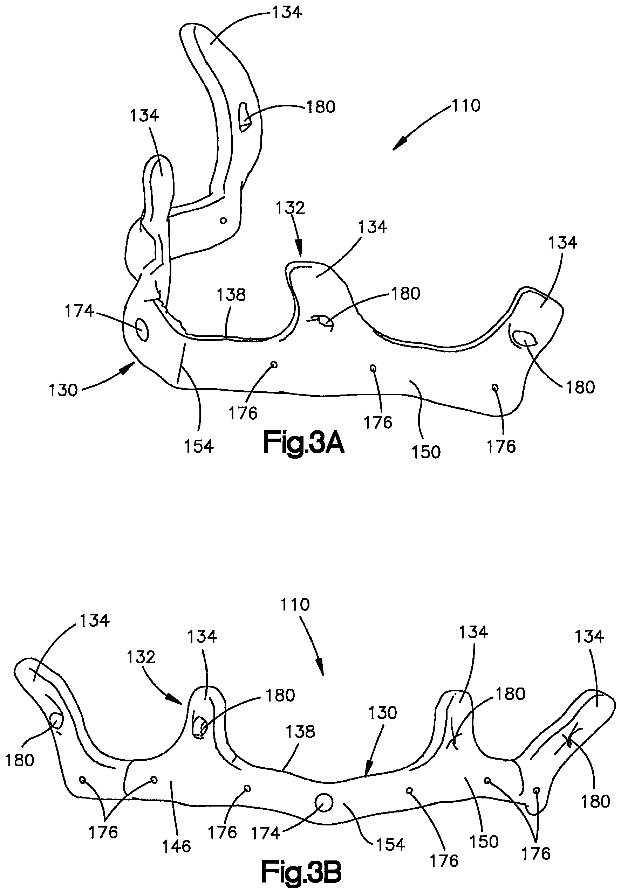

As shown in Figs. 3A-3D, the osteotomy guiding implant 110 includes a longitudinal plate member 130 that is elongate and curved in the longitudinal direction L, and a template portion 132 that includes a plurality of fingers/protrusions 134 that extend vertically in the transverse direction T from the longitudinal member 130. Like the implant 10, the osteotomy guiding implant 110 includes an upper edge 138, a bone engaging surface configured to lie substantially flush with the maxilla, and an outer surface opposed to the bone engaging surface. The osteotomy guiding implant 110 and components thereof, can be formed from a variety of biocompatible materials, such as cobalt chromium molybdenum (CoCrMo), titanium, and titanium alloys, stainless steel, ceramics, or polymers such as polyetheretherketone (PEEK), polyetherketoneketone (PEKK), and bioresorbable materials. A coating may be added or applied to the osteotomy guiding implant 110 to improve physical or chemical properties or to provide medications. Examples of coatings include plasma-sprayed titanium coating or Hydroxyapatite.

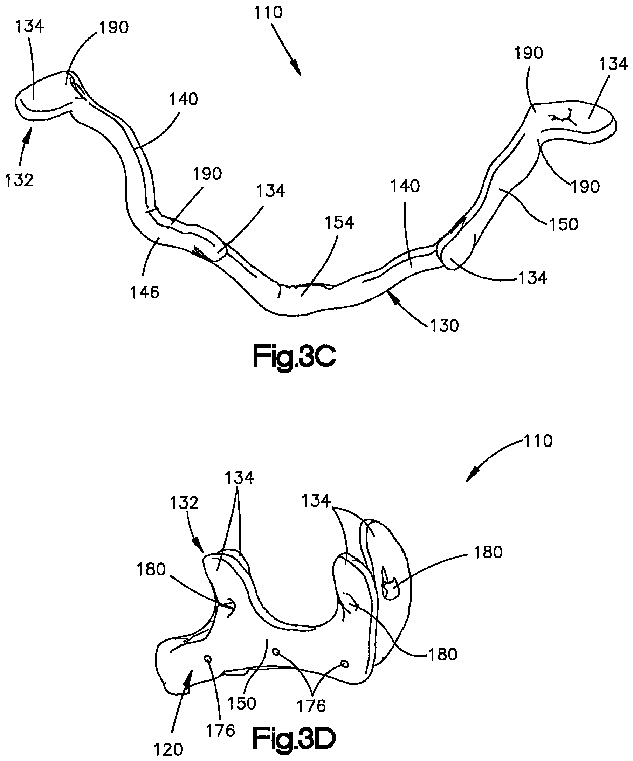

As best shown in Fig. 3C, the longitudinal plate member 130 includes a first portion 146 and a second portion 150 that extend in opposite directions from a central juncture 154. Each portion 146 and 150 curves in the lateral direction A as it extends longitudinally. Therefore, as shown in Fig. 3C, the longitudinal member 130 is curved such that it forms generally a C-shaped structure similar to the bone fixation implant 10. Furthermore, as best shown in Figs. 3B and 3D, the first portion 146 and the second portion 150 each angle up in the transverse direction T as they extend longitudinally. The curvature and shape of the longitudinal member 130 should be configured to correspond to the shape of the maxilla 14.

As shown in Fig. 3B, the longitudinal plate member 130 further includes a reference hole 174 that extends through the plate member 130 proximate to the central juncture 154 from the outer surface to the bone engaging surface. The osteotomy guiding implant 110 may initially be fastened to the maxilla 14 by inserting a fixation element through the reference hole 174 and into the maxilla 14. The fixation element inserted into the reference hole 174 may be temporary and is utilized while a surgeon correctly aligns the implant 110 so that an osteotomy guide may be created.

As shown in Figs. 3A-3D, the longitudinal plate member 130 defines a plurality of apertures or holes 176. As best shown in Fig. 3B, the embodiment illustrated includes three holes 176 in each portion 146 and 150. The holes 176 are spaced apart and provide a template for the surgeon to drill pre-holes into the maxilla 14 that will align with the holes 58 defined by the longitudinal member 30 of the bone implant 10. Therefore, the surgeon will know where to secure the bone implant 10 to the first part 18 of the maxilla 14 after the osteotomy is performed by aligning the holes 58 of the bone implant 10 with the pre-drilled holes. Though it should be understood that in some cases, the longitudinal plate member 130 does not have the apertures 176, and thus the pre-drilled holes are not required to properly align the bone implant 10.

As shown in Figs. 3A-3D, the osteotomy guiding implant's fingers 134 extend up from the upper edge 138 of the longitudinal member 130. In particular, two fingers 134 extend from each of the first and second portions 146, and 150 of the longitudinal member 130. However, it should be understood that any number of fingers 134 may extend up from the first and second portions 146, and 150.

As best shown in Fig. 3B, the fingers 134 are spaced apart along the longitudinal member 130 and extend substantially perpendicularly relative to the point on the longitudinal member 130 from where they extend. That is, the longitudinal member 130 is non-linear and will define tangents at different points along its edge 138. Therefore, the fingers 134 extend perpendicular with respect to a tangent taken at the point on the edge 138 from which the finger 134 extends. Though it should be understood that the fingers 134 do not have to extend perpendicularly and may extend at an angle relative to the longitudinal member 130.

As shown in Fig. 3B, each finger 134 of the osteotomy guiding implant 110 defines an aperture or hole 180. The holes 180 are configured to receive a drill bit so that guide holes may be drilled into the maxilla 14 to thereby define a guide path along which the osteotomy may be performed. As shown, the holes 180 of the fingers 134 will be positioned such that the guide path along which the osteotomy will be performed is appropriately located so that the bone fixation implant 10 may securely hold the first part 18 of the maxilla 14 relative to the second part 22. That is, the osteotomy will be located such that the fingers 80 of the bone implant 10 will be long enough to extend across the osteotomy to securely hold the first part 18 of the maxilla relative to the second part 22.

The osteotomy guiding implant 110, and in particular the longitudinal member 130 and the fingers 134, is pre-shaped to correspond to the pre-operative shape, and relative position of the first part 18 and the second part 22 of the maxilla 14. As best shown in Fig. 3C, the longitudinal member 130 and the fingers 134 include several non-linear undulations 190 that correspond to particular portions of the first part 18 and the second part 22 of the maxilla 14.

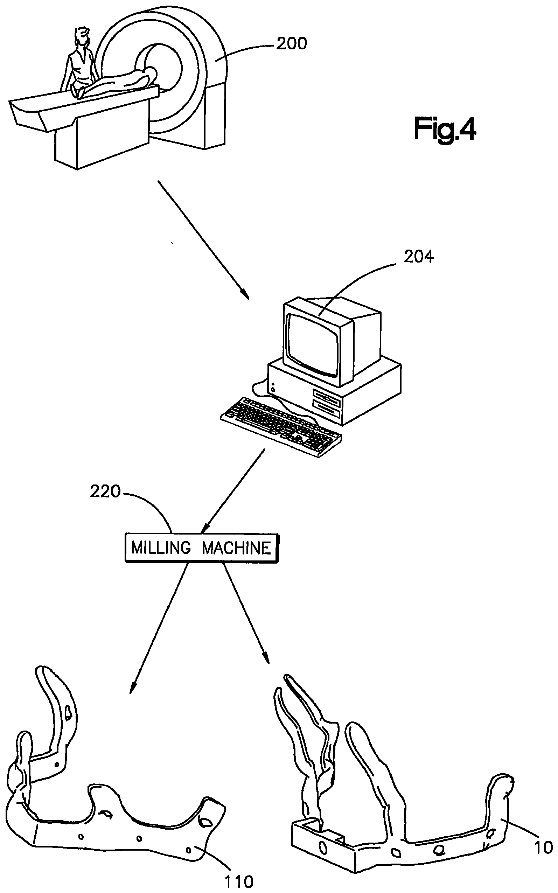

In reference to Fig. 4, both the bone fixation implant 10 and the osteotomy guiding implant 110 are manufactured and shaped pre-operatively. Prior to the orthognathic surgery being performed, a 3-D image of the patient's skull, and in particular the patient's maxilla, such as maxilla 14 is obtained. This may be completed with a CT scanning device 200 or the like, with slices smaller than 1mm preferred, and optimally between 0.2-1 mm. A high resolution for the slices is preferred, since the exact shape of the maxilla 14 should be determined from the CT scan slices. It will be appreciated that other scanning devices 200 besides a CT scanning device may be used so long as they provide three dimensional data corresponding to the shape of the maxilla 14.

Once the 3-D image of the patient's skull/maxilla is obtained, the image is loaded into a computer 204 to create a virtual model of the skull for manipulation by a user such as the surgeon. The computer 204 may be local (same general area as the CT scanning device 200) or remote where the image must be sent via a network. Similarly, the image loaded onto the computer 204 may be manipulated by a user that is working locally or remotely. Typically, however, the image is manipulated remotely by the surgeon who will be performing the orthognathic surgery.

The virtual model of the skull may be manipulated by the surgeon using standard software typical in the art. For example, Mimics, a software commercially available from Materialise, having a place of business in Leuven Belgium, may be used to process and manipulate the virtual model obtained from the CT scanning device 200. The software allows the surgeon to analyze the patient's maxilla and pre-operatively plan the patient's orthognathic surgery including the shape and design of the bone fixation implant a and an osteotomy guiding implant.

Using the virtual model of the patient's skull/maxilla, the surgeon may first make a virtual model of an osteotomy guiding implant, such as the osteotomy guiding implant 110 shown in Figs. 3A-3D. This is accomplished by determining on the virtual model of the skull where the osteotomy is to be performed, and then actually performing a virtual osteotomy on the virtual model. Once the virtual osteotomy is complete, the surgeon can begin making the virtual model of the osteotomy guiding implant 110. At this point, it should be understood that the virtual model of the skull and in particular the maxilla still has its pre-operative shape and position. Therefore, the longitudinal plate member 130 and the fingers 134 of the osteotomy guiding implant 110 that is being made will correspond to the pre-operative shape of the patient's maxilla. The holes 180 that are formed in the fingers 134 of the osteotomy guiding implant 110 will be made in the virtual model to correspond to the virtual osteotomy that was performed on the virtual model of the skull. Therefore, the osteotomy guiding implant 110 manufactured using the virtual model, will define holes 180 that create a guide path for the surgeon to follow while performing the osteotomy. In this way, the actual osteotomy performed on the patient will match the virtual osteotomy that was performed on the virtual model.

After the virtual model of the osteotomy guiding implant 110 is complete, the surgeon or other operator may manipulate the first part 18 (the cut off portion) of the virtual model of the maxilla 14 from a first undesired position to a second desired position. Once the first part 18 is positioned and the virtual model portrays the post-operative shape and position of the patient's maxilla, as approved by the surgeon, a virtual model of a bone fixation implant, such as the bone fixation implant 10 shown in Figs. 2A-2D, can be made. At this point, it should be understood that the virtual model of the skull and in particular the maxilla has a post-operative shape and position. Therefore, the longitudinal plate member 30 and the fingers 80 of the bone fixation implant 10 that is being made will correspond to the post-operative shape of the patient's maxilla.

The virtual models of the osteotomy guiding implant 110 and the bone fixation implant 10 may be downloaded or transferred from the computer 204 to a CAD/CAM milling machine 220 or the like. The milling machine 220 will machine the osteotomy guiding implant 110 and the bone fixation implant 10 out of any desired material. Once the osteotomy guiding implant 110 and the bone fixation implant 10 have been manufactured, the surgeon may begin the orthognathic surgery on the patient.

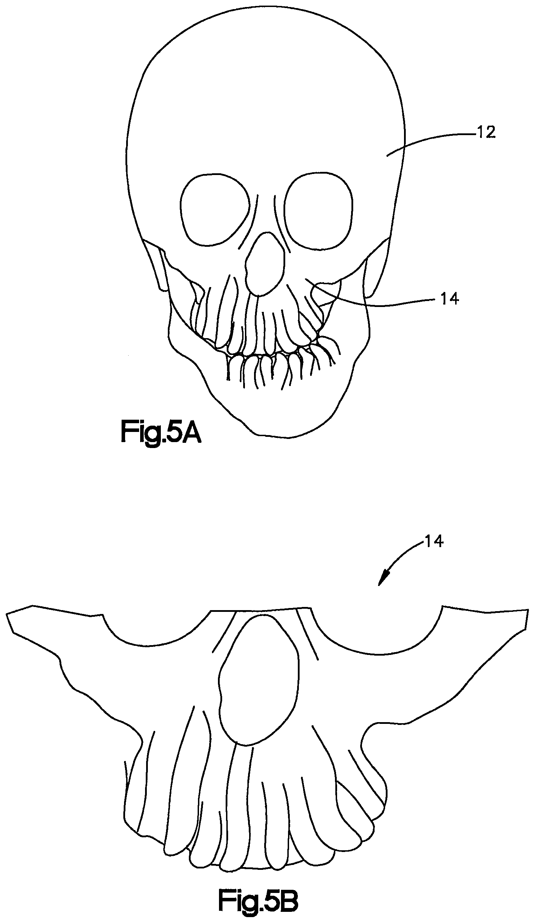

Figs. 5A-5J show an example method of performing an orthognathic surgery using the osteotomy guiding implant 110 and the bone fixation implant 10. It should be understood that prior to the surgery, the osteotomy guiding implant 110 and the bone fixation implant 10 are pre-shaped to substantially correspond to the individual patient's maxilla. Fig. 5A shows an example skull 12 having a maxilla 14 that needs to be repositioned. Fig. 5B is a detailed view of the maxilla 14 shown in Fig. 5A. As shown, the maxilla 14 at this point has a pre-operative shape. An osteotomy is to be performed on the maxilla 14 to thereby separate the maxilla 14 into a first part 18 and a second part 22, so that the first part 18 can be repositioned, as will be described below.

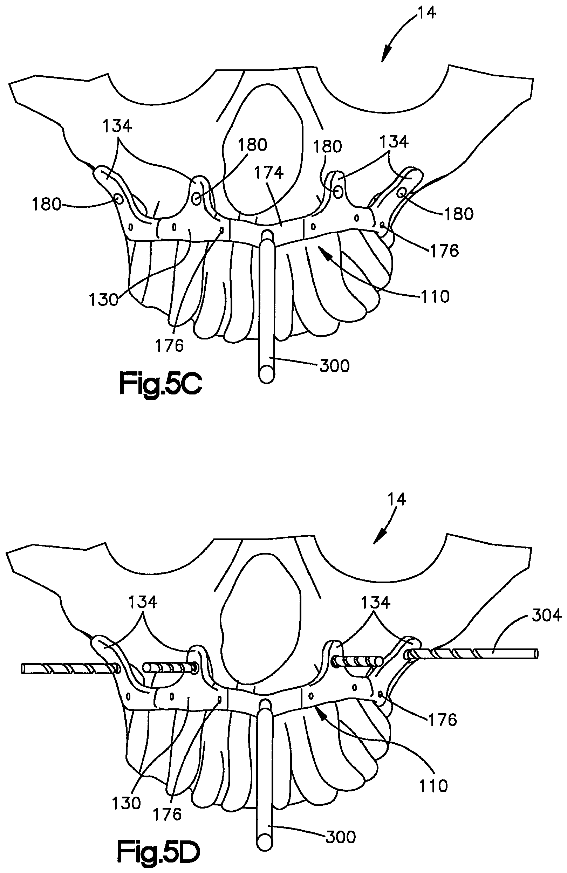

As shown in Fig. 5C, the osteotomy guiding implant 110 may be placed onto the maxilla 14. As stated before, the osteotomy guiding implant 110 is pre-shaped to correspond to the pre-operative shape of the maxilla 14, and therefore will lie flush against the maxilla 14. In other words both the longitudinal plate member 130 and the fingers 134 will be pre-shaped to correspond to the pre-operative shape of the maxilla 14. Once properly positioned, the osteotomy guiding implant 110 may be temporarily affixed to the maxilla 14 by inserting a screw 300 into the reference hole 174 of the osteotomy guiding implant 110 and screwing it into the maxilla 14 with a driver 300.

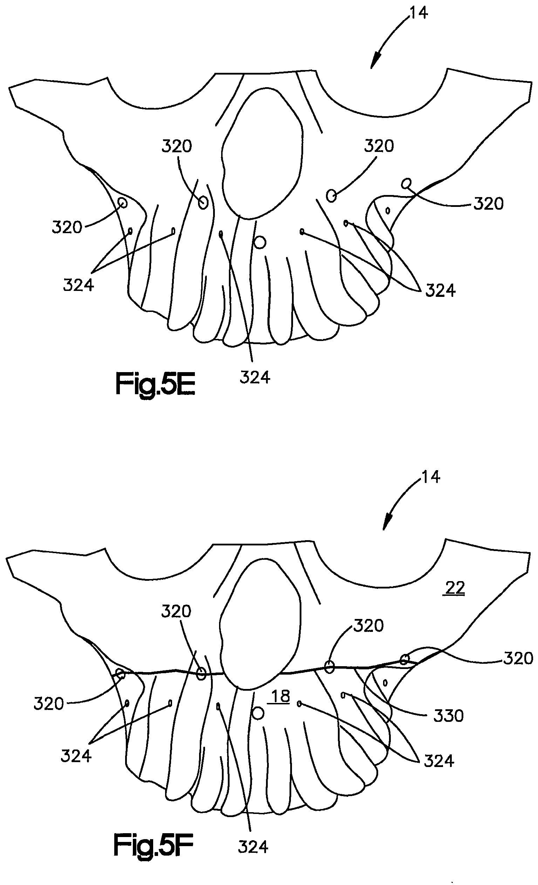

As shown in Fig. 5D, the surgeon may then drill holes into the maxilla 14 using a drill bit 304. As shown, the drill bit 304 may be inserted into the holes 180 defined by the fingers 134 of the osteotomy guiding implant 110. As stated before, the holes 180 are pre-planned and positioned so that the surgeon can create a cutting path for the surgeon to follow while performing the osteotomy. For example, as shown in Fig. 5E, four holes 320 are drilled into maxilla 14 using the osteotomy guiding implant 110. While four holes 320 are shown, it should be understood that the osteotomy guiding implant 110 may be configured so that any number of holes 320 may be made using the osteotomy guiding implant 110. For example, the osteotomy guiding implant 110 may be made to have six fingers 134 so that six holes 320 may be made in the maxilla.

As shown in Fig. 5D, drill bit 304 or another drill bit may be inserted into holes 176 defined by the longitudinal member 130 of the osteotomy guiding implant 110. As shown in Fig. 5E, six holes 324 are drilled into the maxilla 14 using the osteotomy guiding implant 110. While six holes 324 are shown, it should be understood that the osteotomy guiding implant 110 may be configured so that any number of holes 324 may be made using the osteotomy guiding implant 110. The holes 324 will act as a guide for the surgeon to properly place the bone implant 10 to the maxilla. To ensure that bone implant 10 will be securely affixed to the maxilla 14, the holes 324 are smaller than the holes 58 defined by the bone implant 10. Thus when a screw is affixed the threads of the screw will grab a portion of the bone.

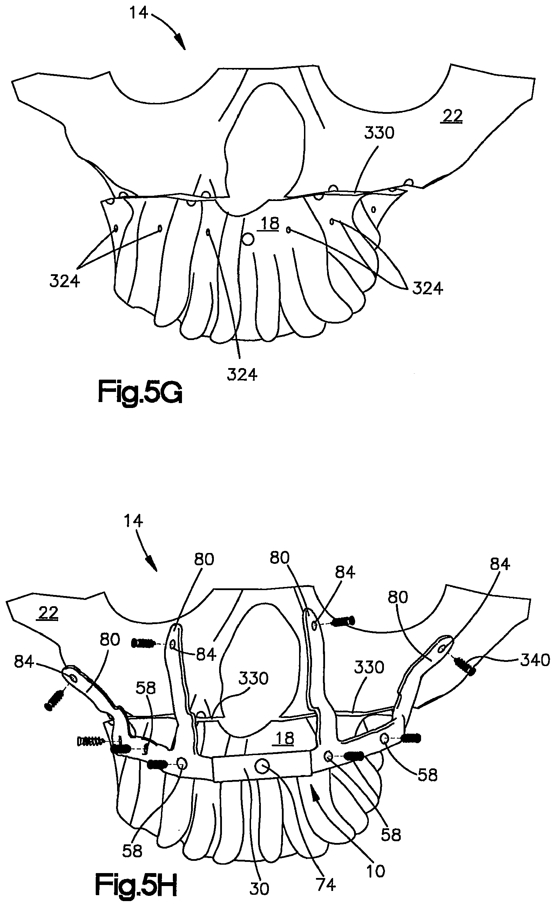

As shown in Fig. 5F, the osteotomy guiding implant 110 may be removed and the surgeon may perform an osteotomy 330 on the maxilla 14 along the cutting path created by the holes 320. In the embodiment illustrated, the cutting path extends from one hole 320 to an adjacent hole 320 until the osteotomy is complete. As shown in Fig. 5G, the osteotomy 330 separates the maxilla into a first part 18 and a second part 22. While the second part 22 remains intact with the skull, the first part 18 is free to be repositioned by the surgeon, for example as shown in Fig. 5G.

Once the first part 18 of the maxilla 14 is repositioned, the bone fixation implant 10 may be placed onto the maxilla 14. As stated before, the bone fixation implant 10 is pre-shaped to correspond to the post-operative shape of the maxilla 14, and therefore will lie flush against the maxilla 14 even after the first part 18 of the maxilla 14 has been repositioned. In other words both the longitudinal plate member 30 and the fingers 80 of the bone plate 10 will be pre-shaped to correspond to the post-operative shape of the maxilla 14. Once properly positioned, the bone fixation implant 10 may be temporarily affixed to the maxilla 14 by inserting a screw into the reference hole 74 of the bone fixation implant 10 and screwing it into the maxilla 14 with the driver 300. In most cases the reference hole 74 of the bone fixation implant 10 will line up with the hole created in the maxilla 14 by the screw that was used to temporarily affix the osteotomy guiding implant 110 to the maxilla 14.

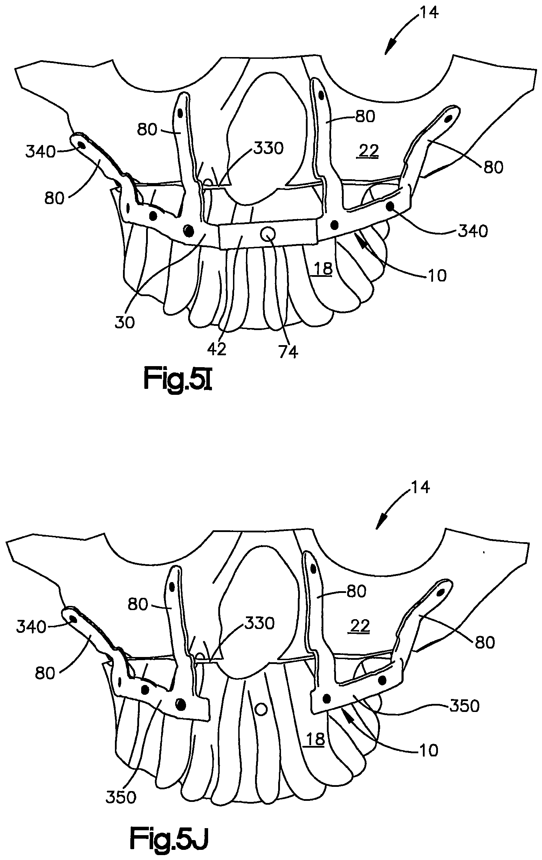

As shown in Figs. 5H and 5I, a plurality of screws 340 may be inserted into the holes 58 and the holes 84 of the bone fixation implant 10. As shown, the fingers 80 of the bone fixation implant 10 are affixed to the second part 22 of the maxilla 14 with the screws 340, and the longitudinal member 30 of the bone fixation implant 10 is affixed to the first part 18 of the maxilla 14 with the screws 340. Therefore, the bone fixation implant 10 is affixed to the maxilla 14 on either side of the osteotomy 330.

As shown in Fig. 5J, the bridge member 42 may then be removed from the bone fixation implant 10 thereby separating the bone fixation implant 10 into two separate parts 350. In this way, the bone fixation implant 10 may be considered to be a single bone fixation implant 10 that is configured to be separated into two separate implant segments or sections after the bone fixation implant 10 has been affixed to bone. As stated before, the bridge member 42 may be removed by either snapping it away or by using pliers or snips to cut the bridge member away at the junctions 54. It should be understood, however, that the bridge member 42 may be removed using any method known in the art.

Once the bridge member 42 is removed, the bone fixation implant 10 is completely installed. Therefore, the surgery may be completed, and the implant 10 may either remain within in the patient or be removed at a later time.

It should be understood that the bone fixation implant 10 and the osteotomy guiding implant 110 may be sold separately or as a kit. It should be understood, however, that the osteotomy guiding implant 110 and bone fixation implant 10 may be manufactured and delivered at different times even though they are part of the same kit. The kit may also include all of the fixation elements required to affix the bone fixation implant 10 to the maxilla 14 as well as any tools required to complete the procedure.

It will be appreciated by those skilled in the art that this invention is not limited to the particular embodiments disclosed, but it is intended to cover modifications within the scope of the present invention as defined by the appended claims.

## Claims

1. A bone fixation implant (10) for use in orthognatic surgery configured to be separated into at least two separate bone implant segments, the bone fixation implant comprising:
a plate member (30) having a first portion (46), and a second portion (50) separated by a bridge portion (42) from the first portion (50), the first and second portions (46, 50) each including at least one aperture (58) that is configured to receive a fixation element (340) so as to secure the plate member (30) to a maxilla (14); and
at least one finger (80) extending from each of the first and second portions (46, 50), each finger (80) including at least one aperture (84) that is configured to receive a fixation element (340) so as to secure each finger (80) to the maxilla (14),
**characterized in that** the bridge portion (42) is removable to thereby separate the first portion (46) of the plate member (30) from the second portion (50) of the plate member (30) after the plate member (30) has been secured to the maxilla (14).

2. The bone fixation implant of claim 1, wherein the first portion (46) extends from the bridge portion (42) at a first junction (54), and the second portion (50) extends from the bridge portion (42) at a second junction (54).

3. The bone fixation implant of claim 1 or 2, wherein the first and second junctions (54) are weakened, preferably perforated.

4. The bone fixation implant of any one of claims 1 to 3, wherein (i) the bridge portion (42) comprises a plate (62) and an extension (66) extending from each end of the plate (62), and (ii) the junctions (54) are located at the ends of the extensions (66).

5. The bone fixation implant of claim 4, wherein the bridge portion (42) comprises a centrally located protrusion (70) extending perpendicularly from a bone facing surface of the plate (62).

6. The bone fixation implant of any one of claims 1 to 5, wherein the bridge portion (42) includes a reference hole (74).

7. The bone fixation implant of any one of claims 1 to 6, wherein the fingers (80) are spaced apart along the plate member (30) and extend substantially perpendicularly relative to the plate member (30).

8. The bone fixation implant of any one of claims 1 to 7, wherein the plate member (30) includes several non-linear undulations (78) that correspond to particular surface portions of the maxilla (14).

9. The bone fixation implant of any one of claims 1 to 8, wherein the fingers (80) include several non-linear undulations (90) that correspond to particular surface portions of the maxilla (14).

10. An orthognathic surgical kit comprising:
an osteotomy guiding implant (110) comprising a plate member (130) and defining a plurality of apertures (180), the apertures (180) being arranged to provide a template for pre-osteotomy drilling holes (320) that define a cutting guide path in a maxilla (14) so as to separate a first portion (18) of the maxilla (14) from a second portion (22) of the maxilla (14); and
a bone fixation implant (10) of any one of claims 1 to 9.

11. The surgical kit of claim 10, wherein the plate member (130) of the osteotomy guiding implant (110) is pre-shaped to correspond to the pre-operative shape of the maxilla (14).

12. The surgical kit of claim 10 or 11, wherein (i) the plate member (130) of the osteotomy guiding implant (110) includes a plurality of fingers (134) that extend from the plate member (130).

13. The surgical kit of any one of claims 10 to 12, further comprising a plurality of fixation elements (340).

14. The surgical kit of any one of claims 10 to 13, wherein (i) the plate member (130) of the osteotomy guiding implant (110) and the plate member (30) of the bone fixation implant (10) each include a reference aperture (174, 74), and (ii) the reference apertures (174, 74) each correspond to a common fixation element receiving hole formed in the maxilla (14).

15. The surgical kit of any one of claims 10 to 14, wherein the osteotomy guiding implant (110) further defines a second plurality of apertures (176) that are arranged to provide a template for pre-osteotomy drilling holes (324) that define a position on the maxilla (14) to place and secure the bone fixation implant (10).

## Patentansprüche

1. Knochenfixierimplantat (10) zur Verwendung in der orthognathen Chirurgie, eingerichtet, in zumindest zwei separate Knochenimplantat-Segmente getrennt zu werden, wobei das Knochenfixierimplantat umfasst:
ein Plattenbauteil (30), das einen ersten Teil (46) und einen zweiten Teil (50), getrennt von dem ersten Teil (50) durch einen Brückenteil (42) umfasst, wobei der erste und zweite Teil (46, 50) jeweils zumindest eine Öffnung (58) enthalten, die eingerichtet ist, ein Fixierelement (340) aufzunehmen, um das Plattenbauteil (30) an einem Oberkiefer (14) zu befestigen, und
zumindest einen Finger (80), der sich von jedem des ersten und zweiten Teils (46, 50) erstreckt, wobei jeder Finger (80) zumindest eine Öffnung (84) enthält, die eingerichtet ist, ein Fixierelement (340) aufzunehmen, um jeden Finger (80) am Oberkiefer (14) zu befestigen,
**dadurch gekennzeichnet, dass** der Brückenteil (42) entfernbar ist, um dadurch den ersten Teil (46) des Plattenbauteils (30) vom zweiten Teil (50) des Plattenbauteils (30) zu trennen, nachdem das Plattenbauteil (30) am Oberkiefer (14) befestigt worden ist.

2. Knochenfixierimplantat nach Anspruch 1, wobei sich der erste Teil (46) von dem Brückenteil (42) an einer ersten Verbindungsstelle (54) erstreckt und sich der zweite Teil (50) von dem Brückenteil (42) an einer zweiten Verbindungsstelle (54) erstreckt.

3. Knochenfixierimplantat nach Anspruch 1 oder 2, wobei die erste und zweite Verbindungsstelle (54) geschwächt, vorzugsweise perforiert, sind.

4. Knochenfixierimplantat nach einem der Ansprüche 1 bis 3, wobei (i) der Brückenteil (42) eine Platte (62) und Verlängerungen (66) umfasst, die sich von jedem Ende der Platte (62) erstrecken, und (ii) sich die Verbindungsstellen (54) an den Enden der Verlängerungen (66) befinden.

5. Knochenfixierimplantat nach Anspruch 4, wobei der Brückenteil (42) einen zentral angeordneten Vorsprung (70) umfasst, der sich senkrecht von einer knochenzugewandten Oberfläche der Platte (62) erstreckt.

6. Knochenfixierimplantat nach einem der Ansprüche 1 bis 5, wobei der Brückenteil (42) ein Referenzloch (74) enthält.

7. Knochenfixierimplantat nach einem der Ansprüche 1 bis 6, wobei die Finger (80) entlang des Plattenbauteils (30) beabstandet sind und sich im Wesentlichen senkrecht relativ zum Plattenbauteil (30) erstrecken.

8. Knochenfixierimplantat nach einem der Ansprüche 1 bis 7, wobei das Plattenbauteil (30) mehrere nichtlineare Wellungen (78) enthält, die zu bestimmten Oberflächenteilen des Oberkiefers (14) korrespondieren.

9. Knochenfixierimplantat nach einem der Ansprüche 1 bis 8, wobei die Finger (80) mehrere nichtlineare Wellungen (90) enthalten, die zu bestimmten Oberflächenteilen des Oberkiefers (14) korrespondieren.

10. Set für orthognathe Chirurgie, umfassend:
ein Osteotomieführungsimplantat (110), das ein Plattenbauteil (130) umfasst und eine Mehrzahl von Öffnungen (180) definiert, wobei die Öffnungen (180) angeordnet sind, eine Schablone zum Bohren von Löchern (320) vor der Osteotomie bereitzustellen, die einen Schnittführungspfad in einem Oberkiefer (14) definieren, um einen ersten Teil (18) des Oberkiefers (14) von einem zweiten Teil (22) des Oberkiefers (14) zu trennen; und
ein Knochenfixierimplantat (10) nach einem der Ansprüche 1 bis 9.

11. Chirurgisches Set nach Anspruch 10, wobei das Plattenbauteil (130) des Osteotomieführungsimplantats (110) vorgeformt ist, um zur präoperativen Form des Oberkiefers (14) zu korrespondieren.

12. Chirurgisches Set nach Anspruch 10 oder 11, wobei (i) das Plattenbauteil (130) des Osteotomieführungsimplantats (110) eine Mehrzahl von Fingern (134) enthält, die sich von dem Plattenbauteil (130) erstrecken.

13. Chirurgisches Set nach einem der Ansprüche 10 bis 12, des Weiteren umfassend eine Mehrzahl von Fixierelementen (340).

14. Chirurgisches Set nach einem der Ansprüche 10 bis 13, wobei (i) das Plattenbauteil (130) des Osteotomieführungsimplantats (110) und das Plattenbauteil (130) des Knochenfixierimplantats (110) jeweils eine Referenzöffnung (174, 74) enthalten, und (ii) die Referenzöffnungen (174, 74) jeweils zu einem im Oberkiefer (14) gebildeten gemeinsamen Fixierelement-Aufnahmeloch korrespondieren.

15. Chirurgisches Set nach einem der Ansprüche 10 bis 14, wobei das Osteotomieführungsimplantat (110) des Weiteren eine zweite Mehrzahl von Öffnungen (176) definiert, die angeordnet sind, eine Schablone zum Bohren von Löchern (324) vor der Osteotomie bereitzustellen, die eine Position auf dem Oberkiefer (14) definieren, um das Knochenfixierimplantat (10) zu platzieren und zu befestigen.

## Revendications

1. Implant de fixation osseuse (10) destiné à une utilisation en chirurgie orthognatique configuré pour être séparé en au moins deux segments d'implant osseux séparés, l'implant de fixation osseuse, comprenant :
un élément formant plaque (30) présentant une première portion (46), et une seconde portion (50) séparée par une portion formant pont (42) de la première portion (50), les première et seconde portions (46, 50) incluant chacune au moins une ouverture (58) configurée pour recevoir un élément de fixation (340) de manière à fixer l'élément formant plaque (30) sur un maxillaire (14), et
au moins un doigt (80) s'étendant à partir de chacune des première et seconde portions (46, 50), chaque doigt (80) incluant au moins une ouverture (84) configurée pour recevoir un élément de fixation (340) de manière à fixer chaque doigt (80) sur le maxillaire (14),
**caractérisé en ce que** la portion formant pont (42) est amovible afin de séparer ainsi la première portion (46) de l'élément formant plaque (30) de la seconde portion (50) de l'élément formant plaque (30) après fixation de l'élément formant plaque (30) sur le maxillaire (14).

2. Implant de fixation osseuse selon la revendication 1, dans lequel la première portion (46) s'étend à partir de la portion formant pont (42) au niveau d'une première jonction (54), et la seconde jonction (50) s'étend à partir de la portion formant pont (42) au niveau de la seconde jonction (54).

3. Implant de fixation osseuse selon la revendication 1 ou 2, dans lequel les première et seconde jonctions (54) sont affaiblies, de préférence perforées.

4. Implant de fixation osseuse selon l'une quelconque des revendications 1 à 3, dans lequel (i) la portion formant pont (42) comprend une plaque (62) et une extension (66) s'étendant à partir de chaque extrémité de la plaque (62), et (ii) les jonctions (54) se situent au niveau des extrémités des extensions (66).

5. Implant de fixation osseuse selon la revendication 4, dans lequel la portion formant pont (42) comprend une saillie se situant de manière centrale (70) et s'étendant perpendiculairement à une surface faisant face à l'os de la plaque (62).

6. Implant de fixation osseuse selon l'une quelconque des revendications 1 à 5, dans lequel la portion formant pont (42) inclut un trou de référence (74).

7. Implant de fixation osseuse selon l'une quelconque des revendications 1 à 6, dans lequel les doigts (80) sont espacés le long d'un élément formant plaque (30) et s'étendent en grande partie perpendiculairement par rapport à l'élément formant plaque (30).

8. Implant de fixation osseuse selon l'une quelconque des revendications 1 à 7, dans lequel l'élément formant plaque (30) inclut plusieurs ondulations non linéaires (78) qui correspondent à des portions de surface particulières du maxillaire (14).

9. Implant de fixation osseuse selon l'une quelconque des revendications 1 à 8, dans lequel les doigts (80) incluent plusieurs ondulations non linéaires (90) qui correspondent à des portions de surface particulières du maxillaire (14).

10. Kit chirurgical orthognatique, comprenant :
un implant de guidage d'ostéotomie (110) comprenant un élément formant plaque (130) et définissant une pluralité d'ouvertures (180), les ouvertures (180) étant agencées pour fournir un gabarit pour des trous de forage pour pré-ostéotomie (320) qui définissent un trajet de guidage de coupe dans un maxillaire (14) de manière à séparer une première portion (18) du maxillaire (14) d'une seconde portion (22) du maxillaire (14), et
un implant de fixation osseuse (10) selon l'une quelconque des revendications 1 à 9.

11. Kit chirurgical selon la revendication 10, dans lequel l'élément formant plaque (130) de l'implant de guidage d'ostéotomie (110) est préformé pour correspondre à la forme préopératoire du maxillaire (14).

12. Kit chirurgical selon la revendication 10 ou 11, dans lequel (i) l'élément formant plaque (130) de l'implant de de guidage d'ostéotomie (110) inclut une pluralité de doigts (134) qui s'étendent à partir de l'élément formant plaque (130).

13. Kit chirurgical selon l'une quelconque des revendications 10 à 12, comprenant en outre une pluralité d'éléments de fixation (340).

14. Kit chirurgical selon l'une quelconque des revendications 10 à13, dans lequel (i) l'élément formant plaque (130) de l'implant de guidage d'ostéotomie (110) et l'élément formant plaque (30) de l'implant de fixation osseuse (10) incluent chacun une ouverture de référence (174, 74), et (ii) les ouvertures de référence (174, 74) correspondent chacune à un trou de réception d'élément de fixation commun formé dans le maxillaire (14).

15. Kit chirurgical selon l'une quelconque des revendications 10 à 14, dans lequel l'implant de guidage d'ostéotomie (110) définit en outre une seconde pluralité d'ouvertures (176) agencées pour fournir un gabarit pour des trous de forage pour pré-ostéotomie (324) qui définissent une position sur le maxillaire (14) pour placer et fixer l'implant de fixation osseuse (10).
